# EUROPEAN PATENT SPECIFICATION

(11) **EP 1 399 549 B1**
(45) Date of publication and mention of the grant of the patent: **31.12.2008**
(21) Application number: 02742553.7
(22) Date of filing: 27.06.2002
(51) Int. Cl.: C12N 15/00, C07K 14/435, C12N 15/63, C12N 5/10, C12N 5/12, C07K 16/18, A61K 38/17, A61K 39/395

(54) **ANTICOMPLEMENT POLYPEPTIDE FROM SALIVARY GLANDS OF THE TICK IXODES RICINUS**
ANTIKOMPLEMENT-POLYPEPTIDE AUS DEN SPEICHELDRÜSEN DER ZECKEN IXODES RICINUS
POLYPEPTIDE ANTICOMPLEMENT PROVENANT DE GLANDES SALIVAIRES DE LA TIQUE IXODES RICINUS

(30) Priority: 27.06.2001 EP 01870143; 01.08.2001 US 309746 P
(43) Date of publication of application: 24.03.2004
(73) Proprietor: Henogen S.A., 6041 Charleroi (BE)
(72) Inventor: DAIX, Virginie, B-6900 Marche-en-Famenne (BE); GODFROID, Edmond, B-1020 Brussels (BE); PASTORET, Paul-Pierre, B-1000 Brussels (BE); BOLLEN, Alex, B-1701 Itterbeek (BE); VANDERPLASSCHEN, Alain, B-1480 Tubize (BE)
(74) Representative: Van Malderen, Joëlle
(86) International application number: PCT/BE2002/000108
(87) International publication number: WO 2003/002734

(56) References cited:
- WO-A-00/77198
- WO-A-01/40469
- WO-A-02/26247
- LAWRIE C H ET AL: "IXODES TICKS: SERUM SPECIES SENSITIVITY OF ANTICOMPLEMENT ACTIVITY" EXPERIMENTAL PARASITOLOGY, NEW YORK, NY, US, vol. 93, no. 4, December 1999 (1999-12), pages 207-214, XP001011316 ISSN: 0014-4894
- RIBEIRO J M C: "IXODES DAMMINI: SALIVARY ANTI-COMPLEMENT ACTIVITY" EXPERIMENTAL PARASITOLOGY, NEW YORK, NY, US, vol. 64, no. 3, 1987, pages 347-353, XP000881653 ISSN: 0014-4894
- JG. VALENZUELA ET AL.,: "Purification, cloning, and expression of a novel salivary anticomplement protein from the tick, Ixodes scapularis" J. BIOL. CHEM., vol. 275, no. 25, 23 June 2000 (2000-06-23), pages 18717-18723, XP002182306 cited in the application
- S. DAS ET AL.,: "Salp25D, an Ixodes scapularis antioxidant, is 1 of 14 immunodominant antigens in engorged tick salivary glands" J. INFECT. DIS., vol. 184, no. 8, 15 October 2001 (2001-10-15), pages 1056-1064, XP001022452

### SEQUENCE LISTING

<110> HENOGEN S.A.
   Daix, Virgine
   Godfroid, Edmond
   Pastoret, Paul-Pierre
   Bollen, Alex
   Vanderplassche, Alain
<120> ANTICOMPLEMENT POLYPEPTIDE
<130> P.HENO.02/WO
<140> PCT/BE02/00108
   <141> 2002606627
<160> 4
<170> PatentIn version 3.1
<210> 1
   <211> 555
   <212> DNA
   <213> Ixodes ricinus
<220>
   <221> CDS
   <222> (1).. (555)
   <223>
<400>
   atg aag act gcg ctg acc tgt gca ctt ttg gcg att tca ttt cta gga        48
   Met Lys Thr Ala Leu Thr Cys Ala Leu Leu Ala Ile Ser Phe Leu Gly
   1               5                   10                  15
   agc cag tgt tcg tac agc gag gat ggc gga gag caa gat aca gga gag        96
   Ser Gln Cys Ser Tyr Ser Glu Asp Gly Gly Glu Gln Asp Thr Gly Glu
               20                  25                  30
   aaa agt act aaa gaa gat ctc tac gaa aaa tat tat aga aat gat tct       144
   Lys Ser Thr Lys Glu Asp Leu Tyr Glu Lys Tyr Tyr Arg Asn Asp Ser
           35                  40                  45
   gga ttg tgc ggg gca cag tac agg aat tta agc tat gcg gaa ccc gtt       192
   Gly Leu Cys Gly Ala Gln Tyr Arg Asn Leu Ser Tyr Ala Glu Pro Val
       50                  55                  60
   tac aac tgc acg ctt aga gct ttg ccc cca atc gtg aat aaa acc tgg       240
   Tyr Asn Cys Thr Leu Arg Ala Leu Pro Pro Ile Val Asn Lys Thr Trp
   65                  70                  75                  80
   gag gaa att agg ctt aac att agc aag tcc ata cct gat ttt gta aag       288
   Glu Glu Ile Arg Leu Asn Ile Ser Lys Ser Ile Pro Asp Phe Val Lys
                   85                  90                  95
   ttg atg tgc aac ttc act gtt gct atg cct gac agc ttc tac tta gtt       336
   Leu Met Cys Asn Phe Thr Val Ala Met Pro Asp Ser Phe Tyr Leu Val
               100                 105                 110
   tat acg gag tca aat gga aac tta agc cct gaa gaa gac gaa cag agc       384
   Tyr Thr Glu Ser Asn Gly Asn Leu Ser Pro Glu Glu Asp Glu Gln Ser
           115                 120                 125
   aca agc aaa ggt gaa aac agt gaa aag gtg tct tct gct gca gtt aca       432
   Thr Ser Lys Gly Glu Asn Ser Glu Lys Val Ser Ser Ala Ala Val Thr
       130                 135                 140
   gtt aca act aac cta ata aca aaa gta cag gaa gaa tgc acg gcg aat       480
   Val Thr Thr Asn Leu Ile Thr Lys Val Gln Glu Glu Cys Thr Ala Asn
   145                 150                 155                 160
   ata act gga tgg acc act gaa gcc ccg acc aat ctg gaa cct acg gag       528
   Ile Thr Gly Trp Thr Thr Glu Ala Pro Thr Asn Leu Glu Pro Thr Glu
                   165                 170                 175
   acg cct gaa cct gag gcc atc cca tga                                   555
   Thr Pro Glu Pro Glu Ala Ile Pro
               180
<210> 2
   <211> 184
   <213> Ioxides ricinus
<400> 2
   Met Lys Thr Ala Leu Thr Cys Ala Leu Leu Ala Ile Ser Phe Leu Gly
   1               5                   10                  15
   Ser Gln Cys Ser Tyr Ser Glu Asp Gly Gly Glu Gln Asp Thr Gly Glu
               20                  25                  30
   Lys Ser Thr Lys Glu Asp Leu Tyr Glu Lys Tyr Tyr Arg Asn Asp Ser
           35                  40                  45
   Gly Leu Cys Gly Ala Gln Tyr Arg Asn Leu Ser Tyr Ala Glu Pro Val
       50                  55                  60
   Tyr Asn Cys Thr Leu Arg Ala Leu Pro Pro Ile Val Asn Lys Thr Trp
   65                  70                  75                  80
   Glu Glu Ile Arg Leu Asn Ile Ser Lys Ser Ile Pro Asp Phe Val Lys
                   85                  90                  95
   Leu Met Cys Asn Phe Thr Val Ala Met Pro Asp Ser Phe Tyr Leu Val
               100                 105                 110
   Tyr Thr Glu Ser Asn Gly Asn Leu Ser Pro Glu Glu Asp Glu Gln Ser
           115                 120                 125
   Thr Ser Lys Gly Glu Asn Ser Glu Lys Val Ser Ser Ala Ala Val Thr
       130                 135                 140
   Val Thr Thr Asn Leu Ile Thr Lys Val Gln Glu Glu Cys Thr Ala Asn
   145                 150                 155                 160
   Ile Thr Gly Trp Thr Thr Glu Ala Pro Thr Asn Leu Glu Pro Thr Glu
                   165                 170                 175
   Thr Pro Glu Pro Glu Ala Ile Pro
               180
<210> 3
   <211> 537
   <212> DNA
   <213> Ioxides ricinus
<220>
   <221> CDS
   <222> (1)..(537)
   <223>
<400> 3
   atg agg act gcg ctg acc tgt gca ctt ttg gcg att tca ttt tta gga        48
   Met Arg Thr Ala Leu Thr Cys Ala Leu Leu Ala Ile Ser Phe Leu Gly
   1               5                   10                  15
   agc ccg tgt tcg tcc agc gag gac ggc gga gag cga gag tca gga gtg        96
   Ser Pro Cys Ser Ser Ser Glu Asp Gly Gly Glu Arg Glu Ser Gly Val
               20                  25                  30
   gaa act act aca caa agt ctc tac gaa cat ttt ttt aga aat cat tct       144
   Glu Thr Thr Thr Gln Ser Leu Tyr Glu His Phe Phe Arg Asn His Ser
           35                  40                  45
   gga ttg tgc ggg gca cag tac agg aat tca agc tat gcg gaa ccc gtt       192
   Gly Leu Cys Gly Ala Gln Tyr Arg Asn Ser Ser Tyr Ala Glu Pro Val
       50                  55                  60
   tac aac tgc acc ctt aaa gct ttg ccc cca atc gtg aat caa acc tgg       240
   Tyr Asn Cys Thr Leu Lys Ala Leu Pro Pro Ile Val Asn Gln Thr Trp
   65                  70                  75                  80
   gag gga att agg cat aac att agc agg acc ata cct cag ttt gta agc       288
   Glu Gly Ile Arg His Asn Ile Ser Arg Thr Ile Pro Gln Phe Val Ser
                   85                  90                  95
   ttg atg tgc aac ttg act gtt gtt atg cct gac aac ttc tac tta ctt       336
   Leu Met Cys Asn Leu Thr Val Val Met Pro Asp Asn Phe Tyr Leu Leu
               100                 105                 110
   tat atg ggc gac caa acg tca aac tcc caa aat gag gaa cag agc aca       384
   Tyr Met Gly Asp Gln Thr Ser Asn Ser Gln Asn Glu Glu Gln Ser Thr
           115                 120                 125
   ggc act agc gaa gag tct cgt cct gtg aga gtt aca gaa cag gat ata       432
   Gly Thr Ser Glu Glu Ser Arg Pro Val Arg Val Thr Glu Gln Asp Ile
       130                 135                 140
   aca aaa gca gag gaa gcc tgc acg gcg aat ata act ggt tgg acc act       480
   Thr Lys Ala Glu Glu Ala Cys Thr Ala Asn Ile Thr Gly Trp Thr Thr
   145                 150                 155                 160
   aaa gcc ccg acc acg ctg gca cct atg gag acg tct gaa ctt gag gcc       528
   Lys Ala Pro Thr Thr Leu Ala Pro Met Glu Thr Ser Glu Leu Glu Ala
                   165                 170                 175
   atc gcc tga                                                           537
   Ile Ala
<210> 4
   <211> 178
   <212> PRT
   <213> Ixodes ricinus
<400> 4
   Met Arg Thr Ala Leu Thr Cys Ala Leu Leu Ala Ile Ser Phe Leu Gly
   1               5                   10                  15
   Ser Pro Cys Ser Ser Ser Glu Asp Gly Gly Glu Arg Glu Ser Gly Val
               20                  25                  30
   Glu Thr Thr Thr Gln Ser Leu Tyr Glu His Phe Phe Arg Asn His Ser
           35                  40                  45
   Gly Leu Cys Gly Ala Gln Tyr Arg Asn Ser Ser Tyr Ala Glu Pro Val
       50                  55                  60
   Tyr Asn Cys Thr Leu Lys Ala Leu Pro Pro Ile Val Asn Gln Thr Trp
   65                  70                  75                  80
   Glu Gly Ile Arg His Asn Ile Ser Arg Thr Ile Pro Gln Phe Val Ser
                   85                  90                  95
   Leu Met Cys Asn Leu Thr Val Val Met Pro Asp Asn Phe Tyr Leu Leu
               100                 105                 110
   Tyr Met Gly Asp Gln Thr Ser Asn Ser Gln Asn Glu Glu Gln Ser Thr
           115                 120                 125
   Gly Thr Ser Glu Glu Ser Arg Pro Val Arg Val Thr Glu Gln Asp Ile
       130                 135                 140
   Thr Lys Ala Glu Glu Ala Cys Thr Ala Asn Ile Thr Gly Trp Thr Thr
   145                 150                 155                 160
   Lys Ala Pro Thr Thr Leu Ala Pro Met Glu Thr Ser Glu Leu Glu Ala
                   165                 170                 175
   Ile Ala

## Description

### Field of the invention

The present invention is related to a new anticomplement polypeptide as well as to its use and its preparation process.

### Background of the invention and state of the art

In an immune response, the complement activation leads to the production of inflammatory anaphylatoxins and to the formation of a membrane attack complex leading to the lysis of the invading organism. Endogenous regulators exist to prevent pathology associated with unconfined and inadvertent complement activation.

However, various pathogens have developed biochemical pathways and mechanisms to evade the host complement system.

Ticks are ectoparasites which infest many animals (mammals, birds, reptiles and amphibians) and are present in almost every region of the world. In addition, many of these ticks are vectors of pathogens such as viruses (toghoto virus), bacteria (borrelia burgdorferi, rickettsia spp) or protozoa (trypanosoma sp, babesia sp, theileria sp) that cause host morbidity and in some cases mortality, in particular in humans and livestock animals.

The tick salivary glands play an important role in the accomplishment of the blood meal and the transmission of said pathogens.

The tick is able to feed repeatedly on its natural host and has a salivary anticomplement activity that presumably facilitates feeding.

Valenzuela J. et al. (The Journal of Biological Chemistry, Vol. 275, No. 25, pp. 18717-18723 (2000)) describes the purification, cloning and expression of a novel salivary anticomplement protein from the tick Ixodes scapularis.

The authors state that said molecule behaves as a regulator of complement activation in a manner similar to decay accelerating factor and Factor H, which inhibit complement activation by interacting with the C3 convertase of classical or alternative pathways.

However, said sequence does not present any significant sequence similarity with Factor H or with any other protein available in public domain databases. It would thus appear that said molecule belongs to a new class of complement regulatory molecules, which upon successful expression, would constitute appropriate antigens for anti-tick vaccines and/or new therapeutic agents.

The international patent applications WO01/40469 WO2/26247 and Valenzuela et al (J. Biol. CHEM, vol 275, N°25 p18717-18723 (2000) describes ticks isolated recombinants or synthetic DNA molecules, vector comprising said DNA molecules cell transformed by said vector and polypeptides encoded by said DNA molecules as well as pharmaceutical composition comprising them. However, said DNA molecules are obtained from the tick Ixodes scapularis.

### Aims of the invention

The present invention aims to provide new anticomplement polypeptides and polynucleotides encoding them.

A main aim of the present invention is to provide such peptide(s) and polynucleotide(s) of the same class which inhibit complement through classical and alternative pathways and which are therefore suitable antigenic candidates for vaccination against ticks and for blocking transmission of various micro-organisms, especially pathogenic agents carried by ticks.

A further aim of the present invention is to provide a pharmaceutical composition which may comprise said anticomplement polypeptide(s), polynucleotide(s) or variant(s) thereof for modulating (possibly by a synergetic action with other polypeptides, polynucleotide(s) or variants of the same class) the immune response of a mammal, in particular, for reducing complement activation in pathologies involving undesired complement activation such as, for example, post ischemic myocardial destruction and inflammation.

### Summary of the invention

The present invention is related to a novel class of anticomplement molecules obtained from the tick Ixodes resinus. Said anticomplement molecules presenting an alternative biochemical pathway different from other known (especially human) anticomplement molecules and are preferably selected from the group consisting of the amino acid sequence of the polypeptide SEQ.ID.NO.2 (identified hereafter as molecule IRAC 2) and the amino acid sequence of the polypeptide SEQ.ID.NO.1 (identified hereafter as molecule IRAC 1).

The present invention is also related to molecules which present a homology higher than 85%, 90%, 95% or 98%-99% sequence identity with the amino acid sequence SEQ.ID.NO.2.

The present invention is also related to polynucleotides (nucleic acid molecules encoding said polypeptides and their variants, preferably a polynucleotide which presents more than 80%, 85%, 90%, 95%, 98-99 % or 100% sequence identify with the nucleotide sequence SEQ.ID.NO.2 or its complementary strand.

Preferably, said polypeptides and its encoding polynucleotide correspond to the complete sequence SEQ.ID.NO.2, its active fragments or variants thereof (molecules which present the same or a similar anticomplement activity than the complete sequence).

Fragments or variants of the polypeptide(s) according to the invention are also molecules which present the same anticomplement activity with one or more genetic modifications (such as deletion or addition of one or more amino-acids) in the complete sequences SEQ.ID.NO.2 or, such as naturally occurring allelic variants. Such modifications do not modify the above mentioned percentage of homology or sequence identity.

Said variants are also molecules which present a similar anti-complement activity to the polypeptides according to the invention through the same biochemical pathway and acting similarly upon the same active site.

The polypeptides can be also integrated as "native" protein or are part of a fusion protein or may advantageously include additional amino-acid sequences which contain secretory or leader sequences, prosequences, sequences which elute in purification such as multiple histidinoresidue or an additional sequence for stability during recombinant production (tag His in the C-terminal sequence).

Said polypeptides may comprise also marker sequences which facilitate purification of the fuse polypeptide with a sequence as an hexa-histidine peptide as provided in the PQE vector (Invitrogen Inc.) and described by Gentz et al., Proceeding National Academic of Science of the USA, 1989, Vol. 86, pp. 821-824) or an HA tag or glutathione-S transferase. The corresponding polynucleotide may also contain non-coding 5' and 3' sequences such as transcribed non-translated sequences, splicing and polyadenylation signal and ribosome binding sites.

Another aspect of the present invention is related to a vector comprising the polynucleotide or polypeptide according to the invention, said vector being preferably a plasmid, a virus, a liposome or a cationic vesicle able to transfect a cell and to obtain the expression of said polynucleotide by said cell.

A further aspect of the present invention concerns the cell (prokaryotic or eukaryotic cell) transfected by or comprising said vector.

A further aspect of the present invention is related to an inhibitor directed against the polypeptide according to the invention, a fragment (epitope) thereof or a polynucleotide encoding said polypeptide.

Preferably, said inhibitor is an antibody (monoclonal or polyclonal antibody) or an active hypervariable portion thereof. The inhibitor could be also a specific receptor of a blood cell able to interact specifically with said polypeptide or its epitopes. The inhibitor could be also an antisense RNA or a ribozyme directed against the polynucleotide encoding said polypeptide.

The present invention is also related to the cells (hybridomas) expressing and producing said antibody or an active portion thereof.

A further aspect of the present invention is related to a pharmaceutical composition (including a vaccine) comprising an adequate pharmaceutical carrier (or diluent) and at least one of the various elements according to the invention, especially the polypeptide, the variant(s), the encoding polynucleotide, the vector, the cell transformed by said vector or the inhibitor according to the invention.

Advantageously, said pharmaceutical composition comprises the two polypeptides according to the invention which present unexpectedly a synergetic effect when there are administrated.

Said pharmaceutical composition may comprise also a suitable adjuvant, antioxidant buffer, bacteriostatics and solutions which become biotonic with the blood of the recipient and aqueous and non-aqueous sterile suspensions (which may include suspension agents). The adjuvant used in the pharmaceutical composition is advantageously used for modulating the immune response of a mammal (including a human) in order to improve the characteristic of the pharmaceutical composition according to the invention or to reduce its possible side effects. The suitable pharmaceutical carrier or diluent is selected by the person skilled in the art according to the type of administration to the mammal (oral administration, intravenous administration, intradermal administration, intramuscular administration, peritoneal administration, etc.).

The pharmaceutical composition can be present in a formulation in a unidose or multidose container and may be stored in a freeze dry condition which requires only the addition of a sterile liquid carrier.

Such pharmaceutical carrier could be in solid liquid or gaseous form and the suitable dose of administration and the ratio between the pharmaceutical carrier/active compound, varies according to the number of administration dose(s), the mass of the mammal to be treated and the possible side effects of the compound according to the invention upon said mammal.

The pharmaceutical composition according to the invention could be a therapeutic or prophylactic composition such as a vaccine.

The pharmaceutical composition according to the invention could be a suitable composition for allowing a vaccination against tick or various micro-organisms (viruses, bacteria or protozoa), transmitted by tick.

The present invention is also related to an immunological/vaccine formulation which comprises the polynucleotide according to the invention presented according to the techniques well-known by the person skilled in the art such as the one described by Wolff et al. (Science, Vol. 247, pp. 1465-1468 (1999)).

The pharmaceutical composition according to the invention could be used for treating or preventing any disease or pathogenesis involving an uncontrolled or undesired activation of the complement, such as cardiac related disease (in particular partial or total ischemia, phenomenon of tissue destruction following post-infarctus myocarditis or the genesis of renal tissue destruction due to the presence of circulating immuno-complexes). Such pharmaceutical composition could be also used for developing the inhibition of complement activation for the treatment and/or the prevention of (chronic) inflammations, such as chronic rheumatoid arthritis.

The vaccination against tick could be obtained by inducing a high rejection of tick and/or a neutralisation of the protection conferred by tick saliva to vectors transmitted by tick (viruses, bacteria or protozoa).

A final aspect of the present invention is related to the use of the pharmaceutical composition according to the invention for the manufacture of a medicament in the treatment and/or the prevention of various diseases, especially diseases induced by tick or pathogenous agents transmitted by tick, especially Ixodes ricinus, as well as for the treatment or the prevention of cardiac related diseases or inflammations.

### Definitions

« Polypeptide » refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detained monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslational natural processes or may be made by synthetic methods. Modifications include acetylation, acylation, ADP-ribosylation, amidation, covalent attachment of flavin, covalent attachment of a hem moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-linkings, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino of amino acids to proteins such as arginylation, and ubiquitination. See, for instance, PROTEINS - STRUCTURE AND MOLECULAR PROPERTIES, 2nd Ed., T. E. Creighton, W. H. Freeman and Comany, New York, 1993 and Wolt, F., Posttranslational Protein Modifications: Perspectives and Prospects, pp. 1-12 in POSTTRANSLATIONAL COVALENT MODIFICATION OF PROTEINS, B. C. Johnson, Ed., Academic Press, New York, 1983; Seifter et al., "Analysis for protein modifications and nonprotein cofactors", Meth. Enzymol. (1990) 182 : 626-646 and Rattan et al., "Protein Synthesis: Posttranslational Modifications and Aging", Ann NY Acad Sci (1992) 663 : 48-62.

"Polynucleotide" generally refers to any polyribonucleotide or polydeoxyribonucleotide, which may be unmodified RNA or DNA or modified RNA or DNA. "Polynucleotides" include, without limitation single- and double-stranded DNA, DNA that is a mixture of single- and double-stranded regions, single- and double- stranded RNA, and RNA that is a mixture of single- and double-stranded regions, hybrid molecules comprising DNA and RNA that may be single-stranded or, more typically, double-stranded or a mixture of single- and double-stranded regions. In addition, "Polynucleotide" refers to triple-stranded regions comprising RNA or DNA or both RNA and DNA. The term "Polynucleotide" also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "Polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another reference polypeptide. Generally, differences are limited so that the sequences of the reference polypeptide and the variant are closely similar overall and, in many regions, identical. A variant and reference polypeptide may differ is amino acid sequence by one or more substitutions (preferably conservative), additions and deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis. Variants should retain one or more of the biological activities of the reference polypeptide. For instance, they should have similar antigenic or immunogenic activities as the reference polypeptide. Antigenicity can be tested using standard immunoblot experiments, preferably using polyclonal sera against the reference polypeptide. The immunogenicity can be tested by measuring antibody responses (using polyclonal sera generated against the variant polypeptide) against purified reference polypeptide in a standard ELISA test. Preferably, a variant would retain all of the above biological activities.

"Identity" is a measure of the identity of nucleotide sequences or amino acid sequences. In general, the sequences are aligned so that the highest order match is obtained. "Identify" per se has an art-recognised meaning and can be calculated using published techniques. See, e.g.: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds, Humana Press, New Jersey, 1994; SEQUENCE ANALYSIS IN MOLECULAR BIOLOGY, von Heijne, G., Academic Press, 1987; and SEQUENCE ANALYSIS PRIMER, Gribskov, M. and Devereux, J., eds, M Stockton Press, New York, 1991). While there exist a number of methods to measure identity between two polynucleotide or polypeptide sequences, the term "identity" is well known to skilled artisans (Carillo, H., and Lipton, D., SIAM J Applied Math (1998) 48 : 1073). Methods commonly employed to determine identity or similarity between two sequences include, but are not limited to those disclosed in Guide to Huge Computers, Martin J. Bishop, ed., Academic Press, San Diego, 1994, and Carillo, H., and Lipton, D., SIAM J Applied Math (1988) 48 : 1073. Methods to determine identity and similarity are codified in computer programs. Preferred computer program methods to determine identity and similarity between two sequences include, but are not limited to, GCG program package (Devereux, J., et al., J Molec Biol (1990) 215 : 403). Most preferably, the program used to determine identity levels was the GAP program, as was used in the Examples hereafter.

As an illustration, by a polynucleotide having a nucleotide sequence having at least, for example, 95% "identity" to a reference nucleotide sequence is intended that the nucleotide sequence of the polynucleotide is identical to the reference sequence except that the polynucleotide sequence may include an average up to five point mutations per each 100 nucleotides of the reference nucleotide sequence. In other words, to obtain a polynucleotide having a nucleotide sequence at least 35% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5' or 3' terminal positions of the reference nucleotide sequence or anywhere between those terminal positions, interspersed either individually among nucleotides in the reference sequence or in one or more contiguous groups within the reference sequence.

Fragments of *I. ricinus* salivary gland polypeptides are also included in the present invention. A fragment is a polypeptide having an amino acid sequence that is the same as a part, but not all, of the amino acid sequence of the aforementioned *I. ricinus* salivary gland polypeptides. As with *I. ricinus* salivary gland polypeptide, fragment may be "free-standing" or comprised within a larger polypeptide of which they form a part or region, most preferably as a single continuous region. Representative examples of polypeptide fragments of the invention, include, for example, fragments from about amino acid number 1-20, 21-40, 41-60, 61-80, 81-100, and 101 to the end of the polypeptide. In this context "about" includes the particularly recited ranges larger or smaller by several, 5, 4, 3, 2 or 1 amino acid at either extreme or at both extremes.

Preferred fragments include, for example, truncated polypeptides having the amino acid sequence of the *I. ricinus* salivary gland polypeptides, except for deletion of a continuous series of residues that includes the amino terminus, or a continuous series of residues that includes the carboxyl terminus and / or transmembrane region or deletion of two continuous series of residues, one including the amino terminus and one including the carboxyl terminus. Also preferred are fragments characterised by structural or functional attributes such as fragments that comprise alpha-helix and alpha-helix forming regions, beta-sheet and beta-sheet forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate *I. ricinus* salivary gland protein activity, including those with a similar activity or an improved activity, or with a decreased undesirable activity. Also included are those that are antigenic or immunogenic in an animal or in a human.

It is meant by an anticomplement molecule: a compound which is able to interact with one of the component of the complement system and to block its biological properties.

### Short description of the drawing

Figs. 1 and 2 represent the nucleotide and amino acid sequences of the IRAC1 and IRAC2 sequences.

Fig. 3 represents COS-7 cells transfected by the vector pcDNA3.1 - IRAC 2HA (revelation of Tag HA by indirect immunofluorescent labelling).

Figs. 4 and 5 represent the profile of expression of the polypeptides according to the invention in the mammalian cells following their ORF cloning.

Figs. 6 and 7 represent the anticomplement capacity of supernatant of cells transfected by a construction which express the polypeptides according to the invention.

### Detailed description of the invention

### 1. Identification of the anticomplement polypeptides according to the invention

mRNAs were extracted from salivary glands of female ticks. After the blood meal, the RNAs were purified with oligo-dT cellulose (Fasttrack 2.0 kit Invitrogen, Inc.). A reverse transcription has been made with an oligodT primer.

PCR reactions were made with the following forward primers: 5'-acagcactgaggtttcagag-3' and 5'-cagagcaagggtctaccagcc-3' and an oligo-dT reverse primer.

PCR products were cloned into the vector pGEM-TEasy or pGEM-T (Promega), and the inserts were sequenced. This approach allows the recovering of two consensus sequences encoding homologous proteins. These proteins are hereafter identified as IRAC 1 and IRAC 2 for "Ixodes ricinus anticomplement proteins" 1 and 2 respectively. A percentage of identity of said nucleotide and protein sequence of IRAC 1 and IRAC 2 is presented in the following table 1.

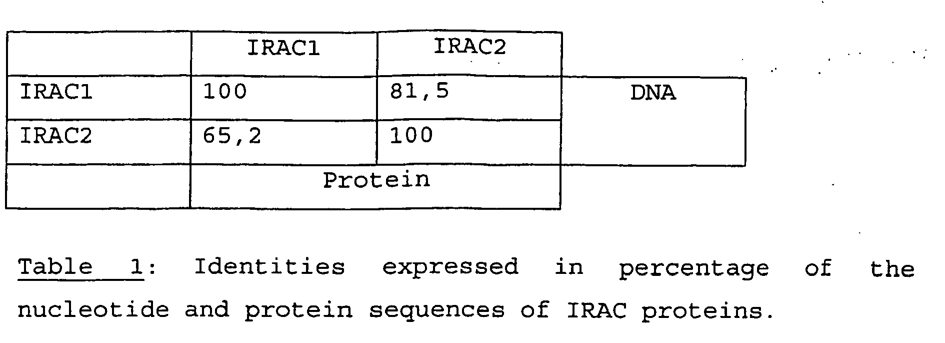

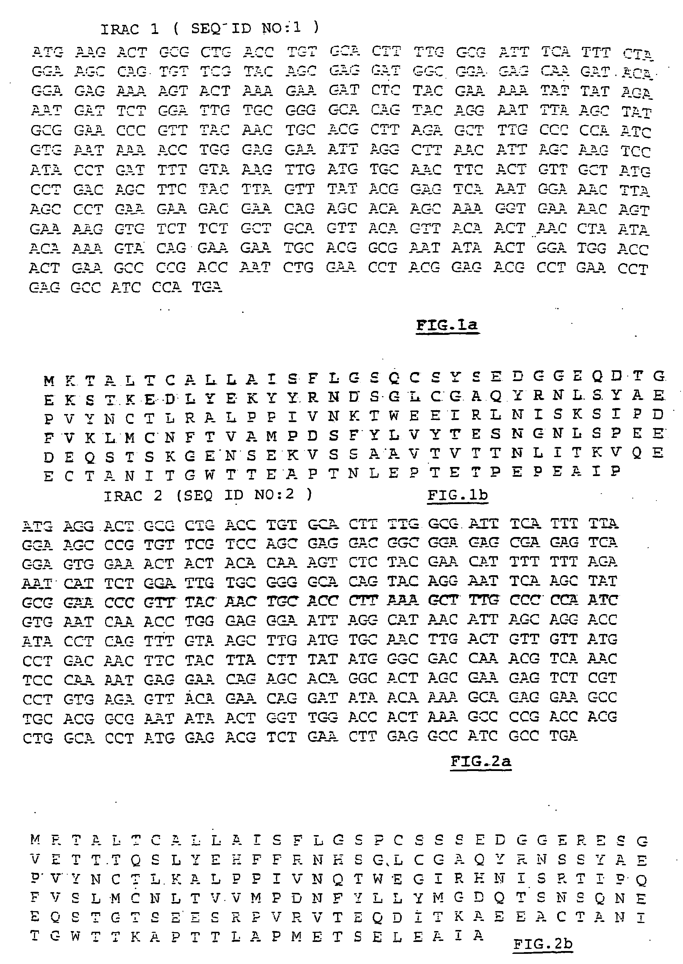

Cloning and expression of the isolated cDNA for IRAC 1 and 2 were achieved by transfection into eukaryotic cells. Corresponding cDNAs were inserted in the vector pcDNA 3.1. Three constructions were made (for each cDNA) in order to obtain a non tagged version of the corresponding proteins as well as two additional C-terminal-tagged versions of the proteins (HA or 6xHis). The transfection of COS-7 cells with said constructions has allowed a high expression of the tagged proteins identified by indirect immuno-labelling (see Fig. 1, a cell expressing the tagged HA IRAC 2 protein).

Since it is possible that the expression of IRAC proteins is toxic to transfected cells, it was proposed to obtain the conditional production of IRAC 1 and IRAC 2 proteins in a cell line (T-Rex System of Invitrogen), after induction by the addition of tetracycline. Four genetic constructions were made using the vector pcDNA4/TO and pcDNA4/TO/myc-His which allow the production of native and tagged proteins (6xHis).

These constructions were transfected into T-Rex-Hela cells (Invitrogen) and stable cell lines were selected with Zeocine.

The tagged proteins were purified thereafter by affinity chromatography upon Ni-chelate columns. Said proteins were thereafter used for animal immunisation in order to produce monoclonal antibodies and polyclonal sera directed against said IRAC 1 and IRAC 2 proteins. These antibodies allow biochemical characterisation of said proteins as well as the identification of cellular and tissular distribution of the said proteins.

Such antibodies were used in experimental passive immunity transfer and were thereafter studied for the capacity to inhibit blood meal of ticks and/or transmission of pathogenic agents (virus, bacteria, protozoa) to a mammalian host.

The proteins according to the invention were also tested in order to check their binding property to the surface of Borrelia burgdorferi and can therefore confer resistance of said bacteria against deleterious effects of the complement produced by the host.

This phenomenon has already been observed with factor H which binds to the surface protein OspE of Borrelia burgdorferi (Hellwage et al., J. Biol. Chem., Vol. 276, pp. 8427-8435 (2001)).

### 2. Mouse immunisation

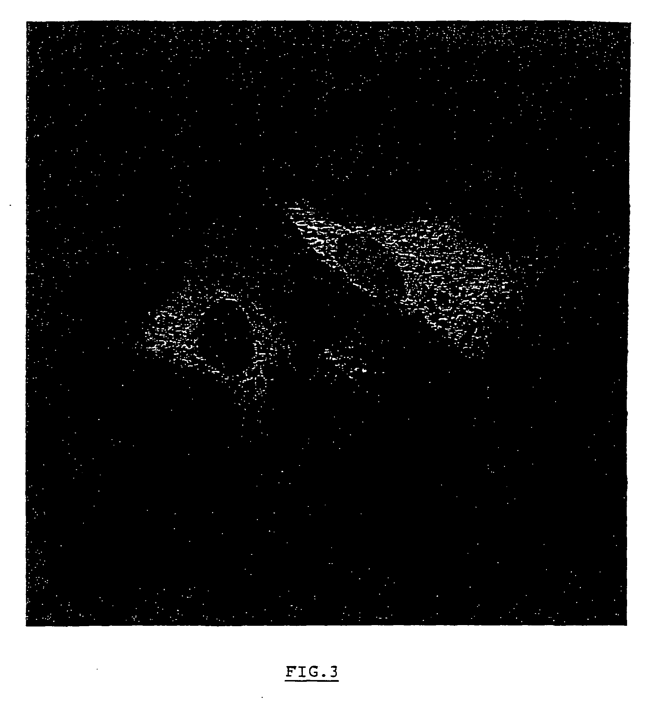

The polypeptides IRAC 1 and IRAC 2 were expressed in mammalian cells with success by cloning the complete open-reading frame (ORF) of said polypeptides (Fig. 3).

The fusion proteins were purified by infinity chromatography and consist in the wild type polypeptide combined with a tag His in the C-terminal extremity.

Said purified proteins were used as antigens for a mouse immunisation.

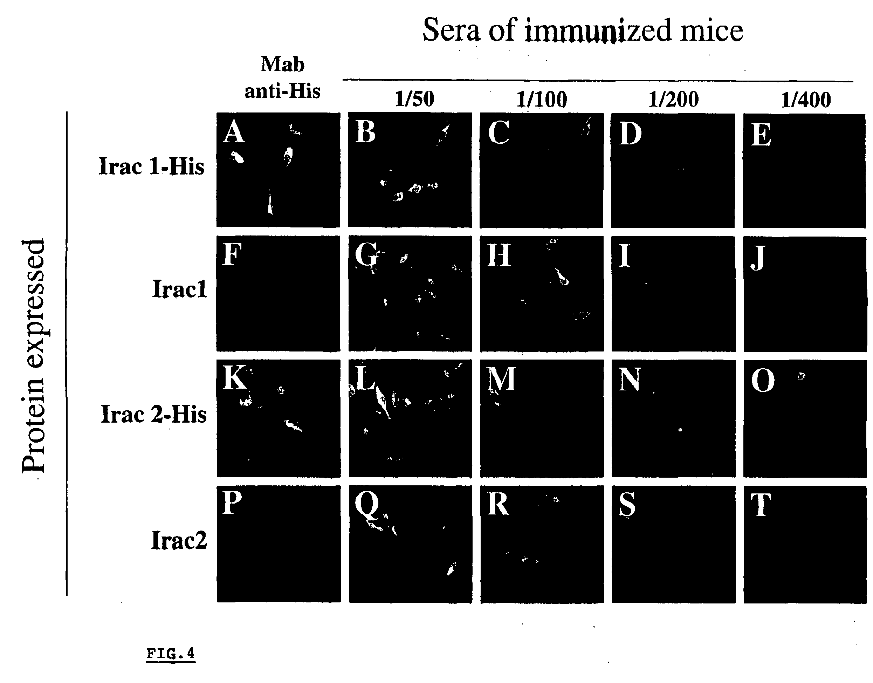

The Sera-analysis following two immunisations demonstrates that said proteins are immunogenic. The analysis of said Sera upon a transfected cell with constructions encoding the wild-type polypeptides according to the invention has demonstrated that the antibodies produced recognise said specific polypeptides (IRAC 1 and/or IRAC 2 proteins) (Fig. 4).

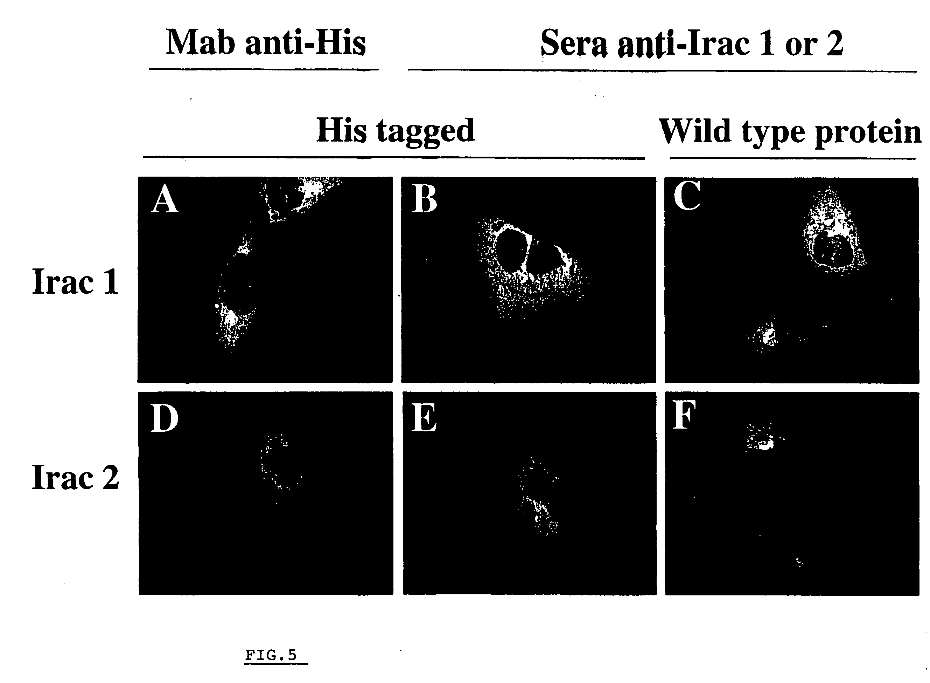

Furthermore, the analysis in cofocal microscopy of transfected cells expressing said polypeptides shows a high expression of said proteins which are presented in the synthesis and excretion system of proteins (Fig. 5). This result demonstrates that polypeptides IRAC 1 and IRAC 2 can be produced under their wild-type or tagged forms and that they are immunogenic. Therefore they can be used for the production of an anti-tick vaccine or in a vaccine to inhibit the transmission of pathogenous agents by ticks.

### 3. Inhibition tests for the characterisation of anticomplement activity

The polypeptides according to the invention are tested for their anticomplement activity. The supernatant of transfected cells with construction expressing the wild-type proteins IRAC 1 and IRAC 2 was tested according to the AH50 proceeding (Current Protocols in Immunology edited by J.E. Coligan et al., National Institute of Health (1992)).

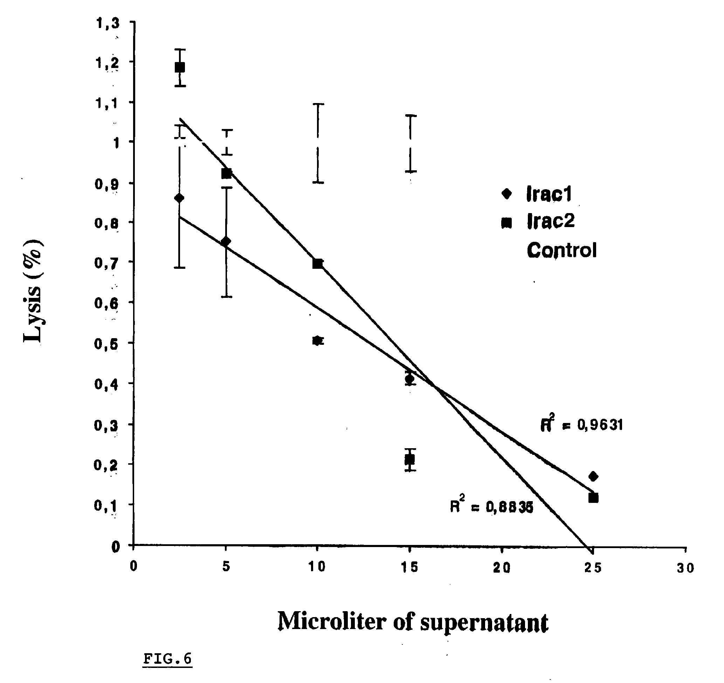

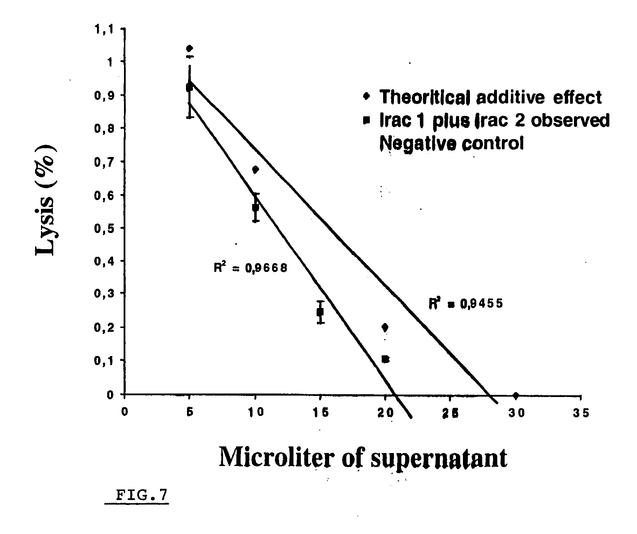

Supernatant of transfected cells with an expression vector without insert, has been used as a negative control. Figs. 6 and 7 show that the two polypeptides according to the invention are able to inhibit complement activity. The polypeptide IRAC 2 seems to present a higher inhibition capacity than IRAC 1 and Fig. 7 shows that a synergetic effect of these two polypeptides can be obtained.

### 4. Identification of the polypeptides synthesis into salivary glands of ticks

Female ticks were selected after four days of blood meal. The salivary glands of said female ticks were recovered and thereafter frozen before being submitted to an immuno-fluorescent detection by the Sera above described. Said experience demonstrates that the polypeptides according to the invention (IRAC 1 and IRAC 2) were synthesised in high concentrations in the salivary glands of ticks following blood meal.

## Claims

1. An anticomplement polypeptide which presents more than 85% identity with the amino-acid sequence SEQ.ID.NO.2.

2. The anticomplement polypeptide according to the claim 1 which presents more than 90% identity with the amino-acid sequence SEQ.ID.NO.2.

3. The anticomplement polypeptide according to the claim 1 or 2 which presents more than 95% identity with the amino-acid sequence SEQ.ID.NO.2.

4. The anticomplement polypeptide according to any of the preceding claims 1 to 3 which presents more than 98-99 % identity with the amino-acid sequence SEQ.ID.NO.2.

5. The anticomplement polypeptide according to any of the preceding claims 1 to 4 which presents the amino-acid sequence SEQ.ID.NO.2.

6. A polynucleotide encoding the anticomplement polypeptide according to any of the preceding claims 1 to 5.

7. A vector comprising the polynucleotide or the anticomplement polypeptide according to any of the preceding claims 1 to 6.

8. A cell transfected or comprising the vector according to the claim 7.

9. An antibody or an active hypervariable portion thereof directed against the polypeptide or the polynucleotide according to any of the preceding claims 1 to 6.

10. A hybridoma cell expressing the antibody or the active portion thereof according to the claim 9.

11. A pharmaceutical composition comprising an adequate pharmaceutical carrier and an element selected from the group consisting of the polypeptide according to any of the preceding claims 1 to 5, the polynucleotide according to the claim 6, the vector according to the claim 7, the cell according to the claim 8 and/or the antibody according to claim 9.

12. Use of the pharmaceutical composition according to the claim 11 for the manufacture of a medicament in the treatment and/or the prevention of diseases induced by tick or pathogenic agents transmitted by ticks, especially but not only Ixodes ricinus.

13. Use of the pharmaceutical composition according to the claim 11 for the manufacture of a medicament in the treatment and/or the prevention of cardiac related diseases or inflammations.

14. Use of the pharmaceutical composition according to the claim 12 for the manufacture of a medicament in the treatment and/or the prevention of cardiac related diseases or inflammations.

## Patentansprüche

1. Antikomplementäres Polypeptid, das mehr als 85 % Identität mit der Aminosäure-Sequenz SEQ.-ID. NR. 2 aufweist.

2. Antikomplementäres Polypeptid nach Anspruch 1, das mehr als 90 % Identität mit der Aminosäure-Sequenz SEQ.-ID. NR. 2 aufweist.

3. Antikomplementäres Polypeptid nach Anspruch 1 oder 2, das mehr als 95 % Identität mit der Aminosäure-Sequenz SEQ.-ID. NR. 2 aufweist.

4. Antikomplementäres Polypeptid nach einem der vorangehenden Ansprüche 1 bis 3, das mehr als 98 - 99 % Identität mit der Aminosäure-Sequenz SEQ.-ID. NR. 2 aufweist.

5. Antikomplementäres Polypeptid nach einem der vorangehenden Ansprüche 1 bis 4, das die Aminosäure-Sequenz SEQ.-ID. NR. 2 aufweist.

6. Polynucleotid, welches das antikomplementäre Polypeptid nach einem der vorangehenden Ansprüche 1 bis 5 kodiert.

7. Vektor, der das Polynucleotid oder das antikomplementäre Polypeptid nach einem der vorangehenden Ansprüche 1 bis 6 umfasst.

8. Zelle, die mit dem Vektor nach Anspruch 7 transfiziert ist oder ihn enthält.

9. Antikörper oder eine aktive, hypervariable Region davon, die gegen das Polypeptid oder das Polynucleotid nach einem der vorangehenden Ansprüche 1 bis 6 gerichtet ist.

10. Hybridomzelle, die den Antikörper nach Anspruch 9 oder die aktive Region davon exprimiert.

11. Pharmazeutische Zusammensetzung, die einen zweckdienlichen pharmazeutischen Träger und ein Grundbestandteil umfasst, das ausgewählt ist aus der Gruppe, die aus dem Polypeptid nach einem der vorangehenden Ansprüche 1 bis 5, dem Polynucleotid nach Anspruch 6, dem Vektor nach Anspruch 7, der Zelle nach Anspruch 8 und/oder dem Antikörper nach Anspruch 9 besteht.

12. Verwendung der pharmazeutischen Zusammensetzung nach Anspruch 11 für die Herstellung von einem Medikament zur Behandlung und/oder der Vermeidung von Erkrankungen, die durch Zecken oder pathogene Wirkstoffe, die durch Zecken übertragen werden, insbesondere, aber nicht ausschließlich Ixodes ricinus, hervorgerufen werden.

13. Verwendung der pharmazeutischen Zusammensetzung nach Anspruch 11 für die Herstellung von einem Medikament zur Behandlung und/oder der Vermeidung von Herzerkrankungen oder -entzündungen.

14. Verwendung der pharmazeutischen Zusammensetzung nach Anspruch 12 für die Herstellung von einem Medikament zur Behandlung und/oder der Vermeidung von Herzerkrankungen oder -entzündungen.

## Revendications

1. Polypeptide anticomplément qui présente plus de 85 % d'identité avec la séquence d'acides aminés SEQ ID N° 2.

2. Polypeptide anticomplément selon la revendication 1 qui présente plus de 90 % d'identité avec la séquence d'acides aminés SEQ ID N° 2.

3. Polypeptide anticomplément selon la revendication 1 ou 2 qui présente plus de 95 % d'identité avec la séquence d'acides aminés SEQ ID N° 2.

4. Polypeptide anticomplément selon l'une quelconque des revendications précédentes 1 à 3 qui présente plus de 98-99 % d'identité avec la séquence d'acides aminés SEQ ID N° 2.

5. Polypeptide anticomplément selon l'une quelconque des revendications précédentes 1 à 4 qui présente la séquence d'acides aminés SEQ ID N° 2.

6. Polynucléotide codant le polypeptide anticomplément selon l'une quelconque des revendications 1 à 5.

7. Vecteur comprenant le polynucléotide ou le polypeptide anticomplément selon l'une quelconque des revendications précédentes 1 à 6.

8. Cellule transfectée ou comprenant le vecteur selon la revendication 7.

9. Anticorps ou partie hypervariable active d'anticorps dirigé(e) contre le polypeptide ou le polynucléotide selon l'une quelconque des revendications précédentes 1 à 6.

10. Cellule d'hybridome exprimant l'anticorps ou sa partie active selon la revendication 9.

11. Composition pharmaceutique comprenant un véhicule pharmaceutique adéquat et un élément choisi dans le groupe consistant en le polypeptide selon l'une quelconque des revendications précédentes 1 à 5, le polynucléotide selon la revendication 6, le vecteur selon la revendication 7, la cellule selon la revendication 8 et/ou l'anticorps selon la revendication 9.

12. Utilisation de la composition pharmaceutique selon la revendication 11 pour la fabrication d'un médicament dans le traitement et/ou la prévention de maladies induites par une tique ou des agents pathogènes transmis par les tiques, en particulier, mais pas exclusivement, *Ixodes ricinus*.

13. Utilisation de la composition pharmaceutique selon la revendication 11 pour la fabrication d'un médicament pour le traitement et/ou la prévention de maladies ou d'inflammations liées au coeur.

14. Utilisation de la composition pharmaceutique selon la revendication 12 pour la fabrication d'un médicament pour le traitement et/ou la prévention de maladies ou d'inflammations liées au coeur.
